# EUROPEAN PATENT SPECIFICATION

(11) **EP 2 730 258 B1**
(45) Date of publication and mention of the grant of the patent: **11.05.2016**
(21) Application number: 12192020.1
(22) Date of filing: 09.11.2012
(51) Int. Cl.: A61F 5/56

(54) **Bite splint**
Aufbissschiene
Plaque occlusale

(43) Date of publication of application: 14.05.2014
(73) Proprietor: Orthotixx Dental AG, 8864 Reichenburg (CH)
(72) Inventor: Unterbrink, Gary, 9495 Triesen (LI)
(74) Representative: Troesch Scheidegger Werner AG

(56) References cited:
- US-A1- 2005 022 824
- US-A1- 2011 100 379
- US-A1- 2011 201 970

## Description

The present invention relates to a bite splint for use in dental field according to the preamble of claim 1.

Anterior bite splints, also called deprogrammers, are known to be used to reduce strain in the muscles of mastication by activating nociceptive trigeminale inhibition. When too much pressure is put on the mandibular incisor teeth of an individual, the neural relaxation reflex is activated and masticatory muscles relax as a result thereof. Thus, tension or migraine headaches due to tense muscles can be reduced or even prevented by wearing a bite splint. Such splints are also frequently helpful to reduce pain in the tempomandibular joints or other head and neck pain associated with craniomandibular dysfunction.

US 2011/201970 A1 discloses an intraoral discluder for use in relieving tension headaches, common migraine headaches, and temporomandibular disorders. Such an apparatus includes a maxillary appliance to receive the maxillary incisors and a separate mandibular appliance to receive and be retained on the mandibulare incisors. Both include T-shaped platforms arranged to face each other in confronting relationship to prevent any occlusive contact between opposing teeth. However such separate small appliances lack of lateral guidance and might therefore shift or slip during use.

An object of the present invention is to provide an improved anterior bite splint.

This objective is achieved by the features given in the characterizing part of claim 1. Further embodiments of the invention are given in dependant claims.

The invention relates to a bite splint having a tunnel-like recess. The recess is defined by two walls being connected to each other via a base element and wherein the first wall protrudes straight downwards from the base element as for instance approximately at 90°. Further, at least a part of an upper surface of the second wall is inclined downwards with respect to an upper surface of the base element. Thereby, pathological load distribution leading to unwanted movement or tipping of teeth is prevented since only axial load is exerted onto the teeth during clenching or pressing.

In one embodiment, a saddle-like platform extends obliquely from the base element in an upwards direction and wherein the second wall is inclined downwards with respect to the upper surface of the platform.
Thereby, a bite contact surface is ensured even for severe malocclusions of Class II Division 1 according to Edward Angle's classification method, where a significant overjet is present. Or in other words, where the mandibular arch is smaller than the maxillary arch by a significant degree.

In one embodiment, a pedestal-like platform protrudes from the base element to the opposite side of the first wall. Thereby, a sufficiently large contact surface for contacting the opposite mandible incisors is ensured even in case the individual exhibits extensive lateral movements of the mandible.

In one embodiment, the bite splint is a maxillary splint or a mandible splint.
Thereby, the bite splint can be adapted to manifold tooth positions, i.e. to standard occlusions but also to anomalous occlusions of a patient.
In one embodiment, an inclination angle between the upper surface of the second wall and the upper surface of the base element is about 20° to 30°, preferably 22° to 28°, preferably 24° to 26° or 25°.
Thereby, pathological load distribution leading to unwanted displacement of teeth is prevented since only axial load is exerted onto the bite contact surface when teeth are clenched.

In one embodiment, a further inclination angle of the tunnel-like recess formed between the first wall and the second wall is about 20° to 40° or about 35°.
Thereby, the bite contact surface is extensive enough, i.e. has sufficient ventral extension to achieve contact between the upper incisor teeth and the bite splint when used as a mandible splint for example. When used as a maxilla splint for example, the upper lip is prevented from becoming trapped by or hanging onto the second wall of the bite splint and thus, prevents dislodgement of the bite splint from its designated wearing position by the perioral musculature.

In one embodiment, the bite splint is dimensioned in its width to be fitted onto at least two adjacent teeth of an individual.
Thereby, retention of the bite splint is ensured when inserted into the mouth of an individual since sufficient undercuts and interproximal contours are available.

In one embodiment, the height of the first wall is about 2cm.
Thereby, the bite splint provides enough material to cover cervical areas of the teeth. Thus, good retention is achieved by providing adequate support of the relining material. Furthermore, the relining material being inserted into the recess is also not visible from the front, and advantage since many relining materials have a tendency to stain.

In one embodiment, at least a part of the second wall is thicker than the first wall.
Thereby, the adaptation of the bite splint can easily be optimized by selectively grinding the internal aspect of the second wall e.g. to adapt the bite splint individually to a teeth of an individual e.g. with malaligned or rotated incisor teeth.

In one embodiment, the second wall is of a wall thickness of about 2mm to 3mm.
This allows adapting the bite splint to manifold tooth positions within a very short time without the need of adding additional structural material for extension of the bite splint.

In one embodiment, the base element or the further base element is of the same thickness as the second wall.

In one embodiment, the width of the first wall is shorter than the width of the second wall with a wing-like element at the endings, such as the lateral aspects of the second wall.

Thereby, the bite splint can quickly be adapted to establish contact with the canine teeth of the maxilla. This allows distribution of loads originating from biting on the bite splint over up to six adjacent teeth.

In one embodiment, an inner surface or the inside of the recess is rough. Thereby, adhesion of relining material to the inner surface of the recess is improved.

In one embodiment, the roughness of the inner surface is 20µm Ra to 40µm Ra.

In one embodiment, the bite splint is semi-transparent or transparent. Thereby, the bite splint easily can be adapted since the dentist can see the teeth through the splint and thus, can verify correct fitting of the splint.

In one embodiment, the edges of the bite splint are rounded such as for instance the outer edges of the bite splint, and wherein bite contact surfaces of the bite splint are smooth. Thereby, reducing the time requirement for finishing and polishing of the bite splint is achieved.

In one embodiment, at least one of the walls comprises a notch at its edge such that the notch demonstrates e.g. a curved notch.

Thereby, the labial frenulum or the lingual frenulum, respectively, can be accommodated without causing discomfort to the individual.

In one embodiment, the bite splint is out of a material of at least one of liquid crystal polymers, polyamide, cyclo-oefin copolymer, polyester copolymer, preferably transparent polyester copolymer.
Thereby, sufficient fracture strength is ensured to resist high bite forces. Furthermore, antagonist teeth in contact with the bite splint are protected from attrition since the materials above mentioned have a low friction coefficient and a low abrasion coefficient. Furthermore, the materials afore-mentioned are resistant to acids, monomers, and cleaning agents. Thus, the bite splint demonstrates an increased life expectancy.

Furthermore, the bite splint according to the present invention allows adaptation in the mouth of the patient without a dental laboratory.

The present invention is further explained with the aid of exemplified embodiments, which are shown in the figures. There is shown in:
fig. 1, schematically, a bite splint according to the present invention in isometric view;
fig. 2, schematically, a bottom view according to figure 1;
fig. 3, schematically, a section A-A according to figure 2;

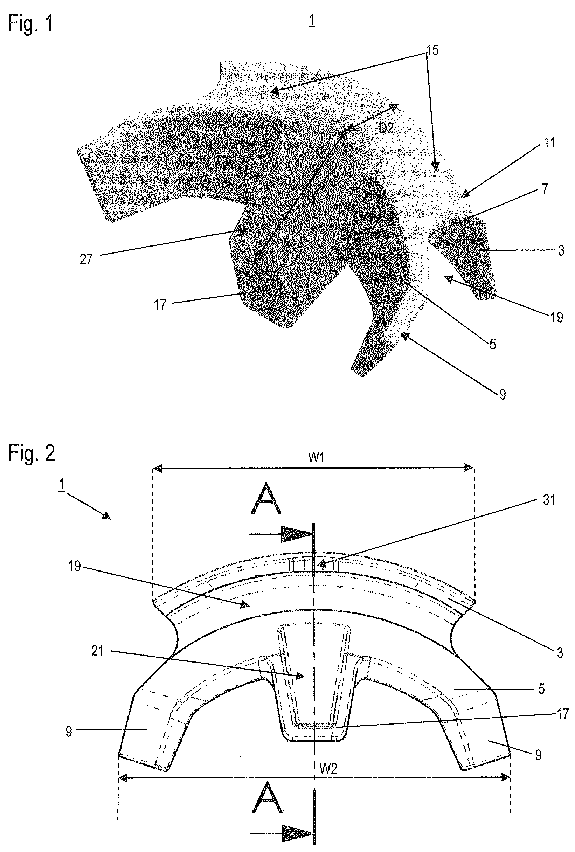

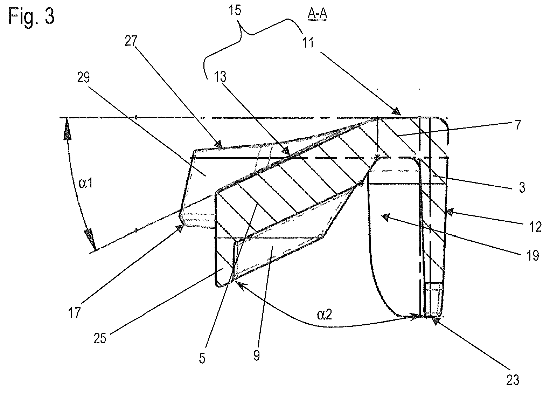

In figure 1, schematically, a bite splint 1 according to the present invention in isometric view is depicted. The tunnel-like bite splint 1 is defined by two walls 3, 5 which are connected to each other via a base element 7. Firstly, the shape of the bite splint 1 is curved according to an average dental arch form. Secondly, a tunnel-like recess 19 of the bite splint 1 is substantially U-shaped. The first wall 3 protrudes downwards from the base element 7 in substantially straight manner such as for instance approximately at 90°. Each of the endings of the second wall 5 comprises a wing-like element 9. The width W1 of the first wall 3 is shorter than the width W2 of the second wall 5 (see figure 2). The wing-like element 9 is arranged bent off from the endings of the first side wall 5 in opposite direction of the first wall 3. An upper surface 11 of the base element 7 and at least a part of an upper surface 13 (see figure 3) of the second wall 5 form a first bite contact surface 15. A region of the upper surface 13 of the second wall 5 is extended towards the opposite side of the first wall 3 forming a platform 17. An upper surface 27 of the platform 17 is a second bite contact surface. The platform 17 is arranged in the middle part of the bite splint 1. The platform 17 is of a pedestal-like or socket-like shape. The depth D1 of the pedestal-like platform 17 is about a third of the depth D2 of the base element 7. The tunnel-like recess 19 for receiving the teeth of an individual is depicted in figure 1. The recess 19 is of a curved shape suitable to receive teeth of nearly any individual. The outer edges of the bite splint 1 are rounded and the bite contact surfaces 15, 27 of the bite splint 1 are smooth. It is also conceivable the all surfaces with the exception of an inner surface of the tunnel-like recess 19 are smooth. The inner surface of the tunnel-like surface is rough.

In figure 2, schematically, a bottom view according to figure 1 is shown. The tunnel-like recess 19 is of a concave shape. The width W1 of the first wall 3 is shorter than the width W2 of the second wall 5. The platform 17 comprises a hollow 21 which is open to a bottom side of the bite splint 1. The first wall 3 demonstrates a rounded notch 31 in its middle of the width W1. The rounded notch 31 serves for reception of the labial frenulum when used a maxillary splint. Each of the endings of the second wall 5 comprises the wing-like element 9. The walls 3, 5 are of a definite thickness. The thickness of the base element 7 is of a definite thickness. The thickness of the first wall 3, of the second wall 5 and of the base element 7 is thick enough such that the dentist can adapt the bite splint according to an individual tooth position by grinding down surplus material of the bite splint. The wing-like element 9 can completely be removed for example, if required. Thus, there is no need to add extension material. Removing portions of the splint when they are not required for the individual situation is fast and simple, adding material to extend the splint is technically difficult and time-consuming.

In figure 3, schematically, a section A-A according to figure 2 is depicted. The first wall 3 protrudes downwards from the base element 7 in almost straight manner, i.e. the upper surface 11 of the base element 7 is nearly perpendicular to the outer side 12 of the first wall 3. The first wall 3 is tapered towards its bottom side 23. The base element 7 comprises the horizontal flattened upper surface 11 being a part of the first bite contact surface 15. At least a further part of the upper surface 13 of the second wall 5 is inclined downwards with respect to the upper surface 11 of the base element 7, the upper surface 13 being also a part of the first bite contact surface 15.

The free ending 25 of the second wall 5 is bent or angled off from the rest of the second wall 5 such that the free ending 25 protrudes downwardly in straight manner such that the free ending 25 forms a wing-like extension. The wing-like extension 25 of the second wall maintains the same vertical angulation, and terminates downward in a straight line. The upper surface 11 of the base element 7 and at least a part of the upper surface 13 of the second wall 5 form the first bite contact surface 15 together. The upper surface 11 of the base element 7 and the upper surface 13 of the second wall 5 are arranged adjacent to each other. An inclination angle α1 between the upper surface 13 of the inclined second wall 5 and the plane upper surface 11 of the base element 7 is about 20° to 30°, preferably 22° to 28°, preferably 24° to 26° or 25°. A further inclination angle α2 of the further tunnel-like recess 19 is formed between the first wall 3 and the free ending 25 of the second wall 5. This further inclination angle α2 is about 20° to 40° or about 35°. The overall width of the bite splint 1 is dimensioned such to cover at least two teeth adjacent to each other, e.g. two incisors. It is also conceivable to utilize more than two teeth, e.g. four incisors teeth or e.g. four incisor teeth including the canine teeth of the maxilla. In this case, the wing-like elements 9 contact the maxillary canine teeth only from the palatal side. The upper side of the respective maxillary canine remains uncovered. This allows providing an individual maxilla bite splint based on one single size.

This allows adaptation to different arch forms without the necessity to have selection of different sizes. This maxillary bite splint is designed for adaptation in the mouth of the patient without a dental laboratory. The pedestal-like platform 17 surmounts the inclined upper surface 13 of the second wall 5, i.e. the upper surface 27 of the pedestal 17 is less inclined compared to the upper surface 13 of the second wall 5. This allows providing a bite splint of a required breaking strength with a high resistance to fracture during use. The upper surface 27 of the pedestal-like platform 17 represents a second bite contact surface. Furthermore, a side wall 29 of the pedestal-like platform 17 is depicted in figure 3. The wing-like element 9 of the second wall 5 is also depicted in figure 3.

## Claims

1. Bite splint (1; 1') having a tunnel-like recess (19; 19') wherein the recess (19; 19') is defined by two walls (3, 5; 3', 5') being connected to each other via a base element (7; 7') and wherein the first wall (3; 3') protrudes straight downwards from the base element (7; 7'), wherein at least a part of an upper surface (13; 13') of the second wall (5; 5') is inclined downwards with respect to an upper surface (11; 11') of the base element (7; 7') **characterized in that** the width (W1) of the first wall (3) is shorter than the width (W2) of the second wall (5) with a wing-like element (9) bent off from each lateral ending of the second wall (5) in opposite direction of the first wall.

2. Bite splint (1) according to claim 1, **characterized in that** a pedestal-like platform (17) protrudes from the base element (7) to the opposite side of the first wall (3).

3. Bite splint (1') according to claim 1 or to claim 2, **characterized in that** a saddle-like platform (17') extends obliquely from the base element (7') in upwards direction and wherein the second wall (5') is inclined downwards with respect to an upper surface (10) of the platform (17').

4. Bite splint (1; 1') according to one of the claims 1 to 3, **characterized in that** the bite splint (1; 1') is a maxillary splint or a mandible splint.

5. Bite splint (1) according to one of the claims 1 to 4, **characterized in that** an inclination angle (α1) between the upper surface (13) of the second wall (5) and the upper surface (11) of the base element (7) is about 20° to 30°, preferably 22° to 28°, preferably 24° to 26° or 25°.

6. Bite splint (1') according to one of the claims 1 to 5, **characterized in that** a further inclination angle (α2; α2') of the tunnel-like recess (19') formed between the first wall (3') and the second wall (5') is about 20° to 40° or about 35°.

7. Bite splint (1; 1') according to one of the claims 1 to 6, **characterized in that** the bite splint (1; 1') is dimensioned in its width to be fitted onto at least two adjacent teeth of an individual.

8. Bite splint (1; 1') according to one of the claims 1 to 7, **characterized in that** the height of the first wall (3; 3') is about 2cm.

9. Bite splint (1; 1') according to one of the claims 1 to 8, **characterized in that** at least a part of the second wall (5; 5') is thicker than the first wall (3; 3').

10. Bite splint (1; 1') according to one of the claims 1 to 9, **characterized in that** the second wall (5; 5') is of a wall thickness of about 2 mm to 3 mm.

11. Bite splint (1; 1') splint according to claim 10, **characterized in that** the base element (7; 7'), is of the same thickness as the second wall (5; 5').

12. Bite splint (1; 1') according to one of the claims 1 to 11, **characterized in that** an inner surface of the recess (19; 19') is rough.

13. Bite splint (1; 1') according to claim 12, **characterized in that** roughness of the inner surface is 20µm Ra to 40µm Ra.

14. Bite splint (1; 1') according to one of the claims 1 to 13, **characterized in that** the bite splint (1; 1') is semi-transparent or transparent.

15. Bite splint (1; 1') according to one of the claims 1 to 14, **characterized in that** edges of the bite splint (1; 1') are rounded and bite contact faces (14; 15; 27) of the bite splint (1; 1') are smooth.

16. Bite splint (1; 1') according to one of the claims 1 to 15, **characterized in that** at least one of the walls (3, 3'; 5, 5') comprises a notch (31) or a groove (31'; 33') at its edge.

17. Bite splint (1; 1') according to one of the claims 1 to 16, **characterized in that** the bite splint (1; 1') is out of a material of at least one of liquid crystal polymers, polyamide, cyclo-olefin copolymer, polyester copolymer, preferably transparent polyester copolymer.

## Patentansprüche

1. Aufbissschiene (1; 1') mit einer tunnelartigen Vertiefung (19; 19') wobei die Vertiefung (19; 19') durch zwei Wände (3,5; 3',5') die miteinander über ein Basiselement (7; 7') verbunden sind gebildet wird, wobei die erste Wand (3; 3') vom Basiselement (7; 7') gerade nach unten ragt und zumindest ein Teil einer oberen Oberfläche (13; 13') der zweiten Wand (5; 5') gegenüber einer oberen Oberfläche (11; 11') des Basiselements (7; 7') nach unten geneigt ist, **dadurch gekennzeichnet, dass** die Spannweite (W1) der ersten Wand (3) kleiner ist als die Spannweite (W2) der zweiten Wand (5) mit einem flügelartigen Element (9) das von jedem seitlichen Ende der zweiten Wand (5) in eine der ersten Wand entgegengesetzten Richtung gebogen ist.

2. Aufbissschiene (1) entsprechend Anspruch 1 **dadurch gekennzeichnet, dass** ein podestartiger Vorsprung (17) vom Basiselement (7) in die der ersten Wand (3) entgegengesetzten Richtung vorspringt.

3. Aufbissschiene (1') entsprechend Anspruch 1 oder 2, **dadurch gekennzeichnet, dass** eine sattelförmige Ebene (17') das Basiselement (7') schräg nach oben verlängert, wobei die zweite Wand (5') bezüglich einer oberen Oberfläche (10) der Ebene (17') nach unten geneigt ist.

4. Aufbissschiene (1; 1') entsprechend einem der Ansprüche 1 bis 3, **dadurch gekennzeichnet, dass** die Aufbissschiene (1; 1') eine Oberkieferschiene oder eine Unterkieferschiene ist.

5. Aufbissschiene (1) entsprechend einem der Ansprüche 1 bis 4, **dadurch gekennzeichnet, dass** ein Neigungswinkel (αA1) zwischen der oberen Oberfläche (13) der Zweiten Wand (5) und der oberen Oberfläche (11) des Basiselements (7) ca. 20° bis 30°, bevorzugt 22° bis 28°, insbesondere bevorzugt 2° bis 26° oder 25° beträgt.

6. Aufbissschiene (1) entsprechend einem der Ansprüche 1 bis 5, **dadurch gekennzeichnet, dass** ein weiterer Neigungswinkel (αA2; αA2') durch die tunnelartige Vertiefung (19') zwischen der ersten Wand (3') und der zweiten Wand (5') gebildet wird und dieser etwa 20° bis 40° oder ca. 35° beträgt.

7. Aufbissschiene (1; 1') entsprechend einem der Ansprüche 1 bis 6, **dadurch gekennzeichnet, dass** die Aufbissschiene (1; 1') bezüglich ihrer Breite so dimensioniert ist, dass sie an zumindest zwei nebeneinander liegenden Zähne einer Person angepasst ist.

8. Aufbissschiene (1; 1') entsprechend einem der Ansprüche 1 bis 7, **dadurch gekennzeichnet, dass** die Höhe der ersten Wand (3; 3') etwa 2 cm ist.

9. Aufbissschiene (1; 1') entsprechend einem der Ansprüche 1 bis 8 **dadurch gekennzeichnet, dass** zumindest ein Teil der zweiten Wand (5; 5') dicker ist als die erste Wand (3; 3')

10. Aufbissschiene (1; 1') entsprechend einem der Ansprüche 1 bis 9, **dadurch gekennzeichnet, dass** die zweite Wand (5; 5') eine Wanddicke von ca. 2 bis 3 mm hat.

11. Aufbissschiene (1; 1') entsprechend Anspruch 10, **dadurch gekennzeichnet, dass** das Basiselement (7; 7'), dieselbe Dicke wie die zweite Wand (5; 5') hat.

12. Aufbissschiene (1; 1') entsprechend einem der Ansprüche 1 bis 11, **dadurch gekennzeichnet, dass** eine innere Oberfläche der Vertiefung (19; 19') rau ist.

13. Aufbissschiene (1; 1') entsprechend Anspruch 12, **dadurch gekennzeichnet, dass** die Rauigkeit der Inneren Oberfläche 20 µm Ra bis 40 µm Ra beträgt.

14. Aufbissschiene (1; 1') entsprechend einem der Ansprüche 1 bis 13, **dadurch gekennzeichnet, dass** die Aufbissschiene (1; 1') semitransparent oder durchsichtig ist.

15. Aufbissschiene (1; 1') entsprechend einem der Ansprüche 1 bis 14, dadurch gekennzeichet, dass die Ecken der Aufbissschiene (1; 1') abgerundet sind und die Bisskontaktflächen (14; 15; 27) der Aufbissschiene (1; 1') glatt sind.

16. Aufbissschiene (1; 1') entsprechend einem der Ansprüche 1 bis 15, **dadurch gekennzeichnet, dass** zumindest eine der Wände (3, 3'; 5, 5') eine Kerbe (31) oder eine Rille (31'; 33') an ihrer Ecke hat.

17. Aufbissschiene (1; 1') entsprechend einem der Ansprüche 1 bis 16, **dadurch gekennzeichnet, dass** die Aufbissschiene (1;) aus zumindest einem der folgenden Materialien besteht: Flüssigkristall Polymere, Polyamide Cyclo-Olefin-Copolymere, Polyester-Copolymere, bevorzugt transparente Polyester-Copolymere.

## Revendications

1. Gouttière occlusale (1, 1') ayant un renfoncement de type tunnel (19, 19'), dans laquelle le renfoncement (19, 19') est défini par deux parois (3, 5 ; 3', 5') raccordées entre elles par un élément de base (7, 7') et dans laquelle la première paroi (3, 3') fait saillie vers le bas par rapport à l'élément de base (7, 7') et dans laquelle au moins une partie d'une surface supérieure (13, 13') de la deuxième paroi (5, 5') est inclinée vers le bas par rapport à une surface supérieure (11, 11') de l'élément de base (7, 7'), **caractérisée en ce que** la largeur (W1) de la première paroi (3) est plus courte que la largeur (W2) de la deuxième paroi (5) avec un élément de type aile (9) rabattu depuis chaque extrémité latérale de la deuxième paroi (5) en direction opposée de la première paroi.

2. Gouttière occlusale (1) selon la revendication 1, **caractérisée en ce qu'**une plateforme de type socle (17) fait saillie depuis l'élément de base (7) vers le côté opposé de la première paroi (3).

3. Gouttière occlusale (1') selon la revendication 1 ou 2, **caractérisée en ce que** une plateforme de type selle (17') s'étend de façon oblique depuis l'élément de base (7') vers le haut et dans laquelle la deuxième paroi (5') est inclinée vers le bas par rapport à une surface supérieure (10) de la plateforme (17').

4. Gouttière occlusale (1, 1') selon l'une des revendications 1 à 3, **caractérisée en ce que** la gouttière occlusale (1, 1') est une gouttière maxillaire ou une gouttière mandibulaire.

5. Gouttière occlusale (1) selon l'une des revendications 1 à 4, **caractérisée en ce que** l'angle d'inclinaison (α1) entre la surface supérieure (13) de la deuxième paroi (5) et la surface supérieure (11) de l'élément de base (7) est d'environ 20° à 30°, de préférence de 22° à 28°, de préférence de 24° à 26° ou de 25°.

6. Gouttière occlusale (1') selon l'une des revendications 1 à 5, **caractérisée en ce qu'**un autre angle d'inclinaison (α2, α2') du renfoncement de type tunnel (19') formé entre la première paroi (3') et la deuxième paroi (5') est d'environ 20° à 40° ou d'environ 35°.

7. Gouttière occlusale (1, 1') selon l'une des revendications 1 à 6, **caractérisée en ce que** la gouttière occlusale (1, 1') est dimensionnée dans sa largeur pour être fixée sur au moins deux dents adjacentes d'une personne.

8. Gouttière occlusale (1, 1') selon l'une des revendications 1 à 7, **caractérisée en ce que** la hauteur de la première paroi (3, 3') est d'environ 2 cm.

9. Gouttière occlusale (1, 1') selon l'une des revendications 1 à 8, **caractérisée en ce qu'**au moins une partie de la deuxième paroi (5, 5') est plus épaisse que la première paroi (3, 3').

10. Gouttière occlusale (1, 1') selon l'une des revendications 1 à 9, **caractérisée en ce que** la deuxième paroi (5, 5') a une épaisseur de paroi d'environ 2 mm à 3 mm.

11. Gouttière occlusale (1, 1') selon la revendication 10, **caractérisée en ce que** l'élément de base (7, 7') a la même épaisseur que la deuxième paroi (5, 5').

12. Gouttière occlusale (1, 1') selon l'une des revendications 1 à 11, **caractérisée en ce que** la surface intérieure du renfoncement (19, 19') est rugueuse.

13. Gouttière occlusale (1, 1') selon la revendication 12, **caractérisée en ce que** la rugosité de la surface intérieure est de 20 µm Ra à 40 µm Ra.

14. Gouttière occlusale (1, 1') selon l'une des revendications 1 à 13, **caractérisée en ce que** la gouttière occlusale (1 ; 1') est semi-transparente ou transparente.

15. Gouttière occlusale (1, 1') selon l'une des revendications 1 à 14, **caractérisée en ce que** les bords de la gouttière occlusale (1, 1') sont arrondis et **en ce que** les faces de contact (14, 15, 27) de la gouttière occlusale (1, 1') sont lisses.

16. Gouttière occlusale (1, 1') selon l'une des revendications 1 à 15, **caractérisée en ce qu'**au moins une des parois (3, 3', 5, 5') comprend une encoche (31) ou une rainure (31', 33') sur son bord.

17. Gouttière occlusale (1, 1') selon l'une des revendications 1 à 16, **caractérisée en ce que** la gouttière occlusale (1, 1') est fabriquée dans un matériau comprenant au moins un élément parmi des polymères à cristaux liquides, polyamide, copolymère de cyclo-oléfine, copolymère de polyester, de préférence un copolymère de polyester transparent.
